# EUROPEAN PATENT SPECIFICATION

(11) **EP 2 158 895 B1**
(45) Date of publication and mention of the grant of the patent: **21.01.2015**
(21) Application number: 09155982.3
(22) Date of filing: 24.03.2009
(51) Int. Cl.: A61K 8/33, A61K 8/34, A61K 8/37, A61Q 13/00, A61K 45/06, A61K 31/01, A61K 31/045, A61K 31/215, A61K 31/22

(54) **Method for energizing human beings**
Verfahren zur Energetisierung von Menschen
Procédé pour énergiser des êtres humains

(30) Priority: 29.05.2008 US 128808
(43) Date of publication of application: 03.03.2010
(73) Proprietor: Symrise AG, 37603 Holzminden (DE)
(72) Inventor: Clothier, James G., Ridgewood, NJ 07450 (US); McDermott, Keith, Bound Brook, NJ 08805 (US); Bellon, Patrice, 92130, Issy Les Moulineaux (FR)
(74) Representative: Fabry, Bernd

(56) References cited:
- WO-A1-02/49600
- JP-A- 9 110 683
- JP-A- 10 117 657
- JP-A- 61 022 019
- JP-A- 63 033 323
- JP-A- 2003 238 986
- US-A- 4 631 146
- US-A- 4 675 318
- US-A1- 2006 240 131
- GHISLAINE BLOCH: "Firmenich finance une étude genevoise pour déceler nos émotions face aux odeurs" LETEMPS.CH, 24 October 2006 (2006-10-24), XP002582550
- Anonymous: "Propriétés des quelques huiles essentielles"[Online] 24 January 2008 (2008-01-24), XP002582551 22 ETOILES.COM Retrieved from the Internet: URL:http://webarchive.org/web/200801240119 07/http://www.22etoiles.com/huilessentiell e/proprietes.htm> [retrieved on 2010-05-17]
- HEUBERGER ET AL.: "East indian sandalwood and alfa-santalol odor increase physiological and self-rated arousal in humans" PLANTA MED, vol. 72, 2006, pages 792-800, XP002582552
- KIECOLT-GLASER ET AL: "Olfactory influences on mood and autonomic, endocrine, and immune function" PSYCHONEUROENDOCRINOLOGY, OXFORD, GB, vol. 33, no. 3, 19 February 2008 (2008-02-19), pages 328-339, XP022489052 ISSN: 0306-4530 DOI: 10.1016/J.PSYNEUEN.2007.11.015
- DATABASE WPI Week 200572 Thomson Scientific, London, GB; AN 2005-691028 XP002610790 & CN 1 628 757 A (SHANDONG HUAQI BIOENGINEERING CO LTD) 22 June 2005 (2005-06-22)
- DATABASE WPI Week 199043 Thomson Scientific, London, GB; AN 1990-324537 XP002610791 & JP 02 232059 A (LION CORP) 14 September 1990 (1990-09-14)
- DATABASE WPI Week 200159 Thomson Scientific, London, GB; AN 2001-530394 XP002610792 & CN 1 299 676 A (XIAMEN MUDANXIANGHUA IND CO LTD) 20 June 2001 (2001-06-20)
- DATABASE CTFA 2006, "Decanal" XP002610793 Database accession no. ID:6226
- Hildbrand et al: "Malonic Acid and Derivatives"[Online] 2005, pages 1-19, XP002610794 Weinheim DOI: 10.1002/14356007.a16_063 Ullmann's Encyclopedia of Industrial Chemistry Retrieved from the Internet: URL:http://onlinelibrary.wiley.com/doi/10. 1002/14356007.a16_063/pdf> [retrieved on 2010-11-19]
- GOUBET N ET AL: "Olfactory familiarization and discrimination in preterm and full term newborns" INFANCY, vol. 3, no. 1, 1 January 2002 (2002-01-01), pages 53-75, XP009141531 WILEY-BLACKWELL, US ISSN: 1525-0008

## Description

### FIELD OF THE INVENTION

The present invention relates to a method for energizing a human being and to administering an energizing (personal care) composition to human beings wherein said composition contains a specific fragrance.

### BACKGROUND OF THE INVENTION

Several currently marketed fragrant cosmetic products claim to have an "energizing" or "vitalizing" benefit to the user. Typically, these products possess fragrances that are purported to deliver these benefits. To support these claims, several methods have been employed to measure the effects of fragrance on physiological parameters with varying degrees of success, and unfortunately, much of the evidence for these purported benefits is the subject of folklore, rather than science.

Further documents that disclose mixtures having an influence on the mood and/or heart rate of human beings encompass:

GHISLANE BLOCH: "Firmenich finance une étude genevoise pour déceler nos émotions face aux odeurs" LE TEMPS CH, 24 October 2006;

"Propriétés des quelques huiles essentielles" 24 January 2008; retrieved from the internet: URL: http//webarchive.org/web/20080124011907/-http://www.22etoiles.com/huilesessentielle/proprieties.htm [retrieved on 17 May 2010].;

HEUBERGER ET AL. "East Indian sandalwood and alfa-santalol odour increases physiological and self-rated arousal in humans "PLANTA MED, vol. 72, pages 792-800 (2006);

KIECOLT-GLASER ET AL.: "Olfactory influences on mood and autonomic endocrines, and immune function" Psychoneuroendocrinology, Vol. 33(3), pages 328-339 (2008);

WO 2002 049600 A1 discloses perfume compositions comprising relaxing components such as tetrahydrogeraniol.

It would be desirable to find other fragrance compositions that are capable of energizing, vitalizing and activating people.

### SUMMARY OF THE INVENTION

This invention relates to a method as defined in claim 1 of energizing a human comprising administering to said human a personal care composition which comprises an effective amount of a fragrance material according to group (a) of the present invention, wherein the personal care composition is capable of energizing said human in terms of an increase of its heart rate (as determined per heartbeat measurement in beats per minute ((bpm)) by about 5% to about 100% based on the heart rate of said human being before administration of said amount of fragrance material.

Various ways to measure the heartbeat of a human being are known in the art, preferably the heartbeat is measured by a device generally known as a heart rate monitor, which allows to measure the heart rate of a human being in real time.

Data collection and analysis of each individual's heart rate is preferably performed using a heart rate sensor, preferably a finger clip (i.e. finger clip attachment for heartbeat measurement) or an ear sensor, more preferably using the Heart Rhythm Monitor using Freeze-Framer 2.0 software (now available as emWave^{®} pc) from HeartMath^{®}, 14700 West Park Avenue, Boulder Creek, California.

For example, US 6,358,201 and US 7,163,512 describe a method for quantifying and analyzing heart rhythms which is called analysis of heart rate variability (HRV). Heart rate variability, which is derived from the electrocardiogram or pulse, is a measure of these naturally occurring beat-to-beat changes in heart rate and is an important indicator of health and fitness. HRV is influenced by a variety of factors, including physical movement, sleep and mental activity, and is particularly responsive to stress and changes in emotional state.

The present invention relates to a non-therapeutic method of energizing a human being comprising the following steps:
- providing an effective amount of a fragrance material comprising or consisting of-1,1-dimethoxy-2,2,5-trimethyl-4-hexene and
- administering said effective amount of fragrance material to said human being in a way that said amount of fragrance material reaches the nasal cavity of said human being,
wherein said administered amount of fragrance material is capable of increasing the heart rate of said human being, preferably by 5% to 100%, more preferably by 5 to 50%, most preferably by 10 to 25%, based on the heart rate of said human being before administration of said amount of fragrance material.

### DETAILED DESCRIPTION OF THE INVENTION

This invention relates to a method of energizing humans beings, in particular as defined above and in the attached set of claims.

In a preferred embodiment the present invention relates to a method wherein said fragrance material comprises at least one additional fragrance selected from the group consisting of tetrahydrogeraniol, methyl dihydro jasmonate, 1-menthol, n-decanal, diethyl malopate, and anethol. In the following text, group (a) corresponds to 1,1-dimethoxy-2,2,5-trimethyl-4-hexene and the fragrance compounds as listed just above.

If mixtures of two fragrance materials of (preferred) group (a) are selected, the ratio by weight of these two materials is preferably in the range of from 5 : 1 to 1 : 5, more preferably in the range of from 3 : 1 to 1 : 3, most preferably in the range of from 2 : 1 to 1 : 2.

In the context of the present invention, the term "administering" refers to fragrances or sensory complexes according to the present invention to be perceived (i) through the nose (orthonasally) or (ii) through the mouth (retronasally), i.e. through the oral cavity, or both.

Thus, the term "administering" includes (i) inhalation of a topically applied personal care composition; (ii) inhalation of the vapours which are released when a personal care composition is dissolved or dispersed in a liquid vehicle such as water, (iii) inhalation of vapors which are released when a personal care composition is dispersed, sprayed, melted or burned, or (iv) retronasal perception of vapours in case the personal care composition is placed into the human oral cavity.

The term "energizing" as used herein refers to invigoration, activation, stimulation, vivication, vitalization or enlivenment of a human being, preferably of an adult having an age from 18 to about 75 years. In particular, a human being is energized when its heart rate is increased by 5 % or more.

The term "effective amount" refers to the amount of the fragrance material which is needed to create the desired energizing response in a human being, and in particular an adult human being.

### SENSORY COMPLEXES

Another aspect of the present invention includes a "sensory complex" capable of energizing a human.

In another preferred embodiment the present invention refers to a method, wherein 1,1-dimethoxy-2,2,5-trimethyl-4-hexene is part of a sensory complex, wherein the sensory complex comprises or consists of:
(i) an effective amount of 1,1-dimethoxy-2,2,5-trimethyl-4-hexen and additionally
(ii) one or more fragrance compounds different from the compounds of group (i) and (iii) as defined below
(iv) one or more essential oils, and/or
(v) one or more cosmetically acceptable diluents, and optionally (iii) one or more fragrance compounds selected from the group consisting of tetrahydrogeraniol, methyl dihydro jasmonate, 1-menthol, n-decanal, diethyl malonate, and anethol.

Typically, the total amount of 1,1-dimethoxy-2,2,5-trimethyl-4-hexene of the sensory complex is from about 1% to about 80%, preferably about 2% to about 70%, more preferably about 4% to about 65%, more preferably about 5% to about 60%, based upon the total weight of the sensory complex.

Typically, the total amount of one or more fragrance compounds (ii) of the sensory complex, preferably selected from group (B) as illustrated below, is from about 0.5% to about 95%, preferably from about 1% to about 75%, based upon the total weight of the sensory complex.

Typically, the total amount of the essential oil portion (iv) of the sensory complex, preferably selected from group (C) as illustrated below, is from about 0.01% to about 30%, preferably from about 0.1% to about 10%, based upon the total weight of the sensory complex.

Typically, the total amount of cosmetically acceptable diluents (v) of the sensory complex, preferably selected from group (D) as illustrated below, is from about 1% to about 80%, preferably from about 5% to about 50%, based upon the total weight of the sensory complex.

In a particular preferred embodiment a "sensory complex" as used in the methods of the present invention consists of
(i) 1,1-dimethoxy-2,2,5-trimethyl-4-hexene,
(ii) one or more fragrance compounds different from compound (i), selected from group (b),
(iii) one or more fragrance compounds selected from the group consisting of tetrahydrogeraniol, methyl dihydro jasmonate, 1-menthol, n-decanal, diethyl malonate, and anethol,
(iv) one or more essential oils, selected from group (c), and
(v) one or more cosmetically acceptable diluents, selected from group (d),
wherein the amounts of groups (i), (ii), (iv) and (v) are in the above given (preferred) weight percentage ranges and the sum of the amounts of groups (i), (ii), (iii), (iv) and (v) is 100%.

### ADDITIONAL FRAGRANCE COMPOUNDS (GROUP B)

The fragrance compounds of the sensory complex are preferably selected from a group (B) consisting of:

**Hydrocarbons,** preferably selected from the group consisting of 3-carene; α-pinene; β-pinene; α-terpinene; γ-terpinene; p-cymene; bisabolene; camphene; caryophyllene, cedrene; farnesene; liminene; longifolene; myrcene; ocimene; valencene; (E,Z)-1,3,5-undecatriene;

**Aliphatic alcohols,** preferably selected from the group consisting of hexanol; octanol; 3-octanol; 2,6-dimethylheptanol; 2-methylheptanol; 2-methyloctanol; (E)-3-hexenol; (E) and (Z)-3-hexenol; 1-octen-3-ol; mixtures of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;

**Aliphatic aldehydes and their acetals,** preferably selected from the group consisting of heptanal; octanal; nonanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanal diethyl acetal; citronellyl oxyacetaldehyde;

**Aliphatic ketones and oximes thereof,** preferably selected from the group consisting of 2-heptanone; 2-octanone; 3-octanone; 2-nonanone; 5-methyl-3-heptanone; 5-methyl-3-heptanone oxime; 2,4,4,7-tetramethyl-6-octen-3-one;

**Aliphatic sulphur-containing compounds,** preferably selected from the group consisting of 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;

**Aliphatic nitriles,** preferably selected from the group consisting of 2-nonenenitrile; 2-tridecenenenitrile; 2,12-tridecenene-nitrile; 3,7-dimethyl-2,6-octadienenitrile; 3,7-dimethyl-6-octenenitrile;

**Aliphatic carboxylic acids and esters thereof,** preferably selected from the group consisting of (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)-and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octynoate; methyl 2-nonynoate; allyl-2-isoamyloxyacetate; methyl-3,7-dimethyl-2,6-octadienoate;

**Acyclic terpene alcohols,** preferably selected from the group consisting of citronellol; geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and formates, acetates, propionates, isobutyrates, butyrates, isovalerates, pentanoates, hexanoates, crotonates, tiglinates, 3-methyl-2-butenoates thereof;

**Acyclic terpene aldehydes and ketones,** preferably selected from the group consisting of geranial; neral; citronellal; 7-hydroxy-3,7-dimethyloctanal; 7-methoxy-3,7-dimethyloctanal; 2,6,10-trimethyl-9-undecenal; geranylacetone; and the dimethyl and diethyl acetals of geranial, neral, 7-hydroxy-3,7-dimethyloctanal;

**Cyclic terpene alcohols,** preferably selected from the group consisting of isopulegol; alpha-terpineol; terpineol-4; menthan-8-ol; menthan-1-ol; menthan-7-ol; borneol; isoborneol; linalool oxide; nopol; cedrol; ambrinol; vetiverol; guaiol; and the formates, acetates, propionates, isobutyrates, butyrates, isovalerates, pentanoates, hexanoates, crotonates, tiglinates, 3-methyl-2-butenoates thereof;

**Cyclic terpene aldehydes and ketones,** preferably selected from the group consisting of menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H)-one; nootkatone; dihydronootkatone; alpha-sinensal; beta-sinensal; acetylated cedarwood oil (methyl cedryl ketone);

**Cyclic alcohols,** preferably selected from the group consisting of 4-tert.-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;

**Cycloaliphatic alcohols,** preferably selected from the group consisting of alpha-3,3-trimethylcyclohexylmethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-l-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;

**Cyclic and cycloaliphatic ethers,** preferably selected from the group consisting of cineol; cedryl methyl ether; cyclododecyl methyl ether; (ethoxymethoxy)cyclododecane; alpha-cedrene epoxide; 3a,6,6,9a-tetramethyl-dodecahydronaphtho[2,1-b]furan; 3a-ethyl-6,6,9a-trimethyldodecahydro-naphtho[2,1-b]furan; 1,5,9-trimethyl-13-oxabicyclo[10.1.0]trideca-4,8-diene; rose oxide; 2-(2,4-dimethyl-3-cyclohexen-1-yl)-5-methyl-5-(1-methylpropyl)-1,3-dioxane;

**Cyclic ketones,** preferably selected from the group consisting of 4-tert.-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert.-pentylcyclohexanone; 5-cyclohexadecen-1-one; 6,7-dihydro-1,1,2,3,3-pentamethyl-4(5H)-indanone; 9-cycloheptadecen-1-one; cyclopentadecanone; cyclohexadecanone;

**Cycloaliphatic aldehydes,** preferably selected from the group consisting of 2,4-dimethyl-3-cyclohexenecarbaldehyde; 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl)-3-cyclohexenecarbaldehyde;

**Cycloaliphatic ketones,** preferably selected from the group consisting of 1-(3,3-dimethylcyclohexyl)-4-penten-1-one; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl-2,6,10-trimethyl-2,5,9-cyclododecatrienyl ketone; tert.-butyl (2,4-dimethyl-3-cyclohexen-1-yl) ketone;

**Esters of cyclic alcohols,** preferably selected from the group consisting of 2-tert.-butylcyclohexyl acetate; 4-tert.-butyl-cyclohexyl acetate; 2-tert.-pentylcyclohexyl acetate; 4-tert.-pentylcyclohexyl acetate; decahydro-2-naphthyl acetate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;

**Esters of cycloaliphatic carboxylic acids,** preferably selected from the group consisting of, allyl 3-cyclohexylpropionate; allyl cyclohexyloxyacetate; methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;

**Aromatic hydrocarbons,** preferably selected from the group consisting of styrene and diphenylmethane;

**Araliphatic alcohols,** preferably selected from the group consisting of benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-l-ol; 4-methoxybenzyl alcohol; 1-(4-isopropylphenyl)ethanol;

**Esters of araliphatic alcohols and aliphatic carboxylic acids,** preferably selected from the group consisting of benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;

**Araliphatic ethers,** preferably selected from the group consisting of 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxin; 4,4a,5,9b-tetrahydro-2,4-dimethylindeno[1,2-d]-m-dioxin;

**Aromatic and araliphatic aldehydes,** preferably selected from the group consisting of benzaldehyde; phenylacetaldehyde; 3-phenylpropanal; hydratropaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert.-butylphenyl)propanal; 3-(4-tert.-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxy-benzaldehyde; 2-methyl-3-(4-methoxyphenyl)propanal; 2-methyl-3-(4-methylenedioxyphenyl)propanal;

**Aromatic and araliphatic ketones,** preferably selected from the group consisting of acetophenone; 4-methylacetophenone; 4-methoxyacetophenone; 4-tert.-butyl-2,6-dimethylacetophenone; 4-phenyl-2-butanone; 4-(4-hydroxyphenyl)-2-butanone; 1-(2-naphthalenyl)ethanone; benzophenone; 1,1,2,3,3,6-hexamethyl-5-indanyl methyl ketone; 6-tert.-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;

**Aromatic and araliphatic carboxylic acids and esters thereof,** preferably selected from the group consisting of benzoic acid; phenylacetic acid; methyl benzoate; ethyl benzoate; hexyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;

**Nitrogen-containing aromatic compounds,** preferably selected from the group consisting of 2,4,6-trinitro- 1,3-dimethyl-5-tert.-butylbenzene; 3,5-dinitro-2,6-dimethyl-4-tert.-butylacetophenone; cinnamonitrile; 5-phenyl-3-methyl-2-pentenenitrile; 5-phenyl-3-methylpentanenitrile; methyl anthranilate; methyl N-methylanthranilate; Schiff bases of methyl anthranilate with 7-hydroxy-3,7-dimethyloctanal; 2-methyl-3-(4-tert.-butylphenyl)propanal or 2,4-dimethyl-3-cyclohexene-carbaldehyde; 6-isopropylquinoline; 6-isobutylquinoline; 6-sec.-butylquinoline; indole; skatole; 2-methoxy-3-isopropylpyrazine; 2-isobutyl-3-methoxypyrazine;

**Phenols, phenyl ethers and phenyl esters,** preferably selected from the group consisting of estragole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;

**Heterocyclic compounds,** preferably selected from the group consisting of 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;

**Lactones,** preferably selected from the group consisting of 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 1,15-pentadecanolide; cis and trans-11-pentadecen-1,15-olide; cis and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

More preferred fragrance compounds of group (B) of the sensory complex are selected from the group consisting of:

| |
|---|
| AGRUMEX^{®} |
| AGRUNITRIL |
| ALDEHYDE C8 |
| ALDEHYDE C9 |
| ALDEHYDE C10 |
| ALDEHYDE C11 UNDECYLENIC |
| ALDEHYDE C12 LAURIC |
| ALDEHYDE C14 SO-CALLED |
| ALLYL AMYL GLYCOLATE |
| ALLYL CAPROATE |
| ALLYL CYCLOHEXYL PROPIONATE |
| ALLYL HEPTOATE |
| ALLYL IONONE |
| AMBROXAN^{®} |
| ANETHOL |
| ANISYL ACETATE |
| BENZALDEHYDE |
| BOURGEONAL^{®} |
| CARVONE L |
| DAMASCENONE |
| DAMASCONE DELTA |
| DIHYDROEUGENOL |
| DIHYDROMYRCENOL |
| DIMETHYL BENZYL CARBINYL ACETATE |
| DIMETHYL BENZYL CARBINYL BUTYRAT |
| ETHYL BUTYRATE |
| ETHYL CAPROATE |
| ETHYL LINALOOL |
| ETHYL MALTOL |
| ETHYL METHYL BUTYRATE-2 |
| ETHYL VANILLIN |
| ETHYLENE BRASSYLATE |
| EUCALYPTOL |
| EVERNYL^{®} |
| FLORAZON^{®} |
| GERANIOL |
| GLOBALIDE^{®} |
| HERBAFLORAT^{®} |
| HERBYL PROPIONATE |
| HEXENOL CIS-3 |
| HEXENYL ACETATE CIS-3 |
| HEXYL ACETATE |
| INDOLE |
| IONONE BETA |
| ISO E SUPER^{®} |
| JASMOPYRANE^{®} |
| LIGUSTRAL^{®} |
| LILIAL^{®} |
| LINALOOL OXIDE |
| LINALYL ACETATE |
| LINALYL ISOBUTYRATE |
| MACROLIDE^{®} |
| MALTOL |
| MELONAL^{®} |
| MENTHONE L |
| ISOMENTHONE |
| MENTHYLACETATE |
| METHYL ANTHRANILATE |
| METHYL HEPTENONE-6,5,2 |
| MUGETANOL^{®} |
| PHENIRAT^{®} |
| PHENOXANOL^{®} |
| PHENYLETHYL ACETATE |
| PHENYLETHYL ALCOHOL |
| PHENYLETHYL ALCOHOL |
| PHENYLETHYLPHENYLACETATE |
| PRENYL ACETATE |
| ROSE OXIDE |
| TETRAHYDROLINALOOL |
| THYMOL |
| UNDECAVERTOL^{®} |
| VANILLIN |

The corresponding chemical names and/or structures of the preferred fragrance compounds of group (B) can be found in H. Surburg and J. Panten, Common Fragrance and Flavor Materials, 5th edition, Wiley-VCH, Weinheim 2006.

### ESSENTIAL OILS (GROUP C)

The essential oils of the sensory complex are preferably selected from group (C) consisting of: amyris oil; angelica seed oil; angelica root oil; aniseed oil; valerian oil; basil oil; bay oil; mugwort oil; benzoin resin; bergamot oil; birch tar oil; bitter almond oil; savory oil; bucco-leaf oil; cabreuva oil; cade oil; calamus oil; camphor oil; cananga oil; cardamom oil; cascarilla oil; cassia oil; cedar-leaf oil; cedarwood oil; cistus oil; citronella oil; lemon oil; coriander oil; costus root oil; cumin oil; cypress oil; davana oil; dill oil; dillseed oil; eucalyptus citriodora oil; eucalyptus oil; fennel oil; fir oil; galbanum oil; geranium oil; grapefruit oil; guaiac wood oil; ginger oil; calamus oil; blue chamomile oil; Roman chamomile oil; carrot-seed oil; cascarilla oil; pine-needle oil; spearmint oil; caraway oil; labdanum oil; lavandin oil; lavender oil; lemongrass oil; lovage oil; distilled lime oil; pressed lime oil; linaloe oil; litsea cubeba oil; bay-leaf oil; mace oil; marjoram oil; mandarin oil; massoi bark oil; ambrette oil; clary sage oil; myristica oil; myrrh oil; myrtle oil; clove leaf oil; clove flower oil; neroli oil; olibanum oil; opopanax oil; orange oil; origanum oil; palmarosa oil; patchouli oil; perilla oil; Peru balsam oil; parsley leaf oil; parsley seed oil; petitgrain oil; peppermint oil; pepper oil; pimento oil; pine oil; pennyroyal oil; rosewood oil; rose oil; rosemary oil; Dalmatian sage oil; Spanish sage oil; sandalwood oil; celery seed oil; spike lavender oil; Japanese aniseed oil; styrax oil; tagetes oil; fir-needle oil; tea-tree oil; turpentine oil; thyme oil; verbena oil; vetiver oil; juniper oil; wormwood oil; wintergreen oil; ylang oil; hyssop oil; cinnamon leaf oil; cinnamon bark oil.

The essential oils of group (C) of the sensory complex are more preferably selected from the group consisting of: cardamom oil, chamomile oil, cinnamon oil, clove oil, geranium oil, ginger oil, grapefruit oil, lemon oil, lemongrass oil, orange oil, peppermint oil, pine oil, rosemary oil, spearmint oil, thyme oil.

### DILUENTS (GROUP D)

The cosmetically acceptable diluents of the sensory complex are preferably selected from group (D) consisting of ethanol, dipropylene glycol, propylene glycol, 1,2-butylene glycol, glycerol, diethylene glycol monoethyl ether, diethyl phthalate, isopropyl myristate, triethyl citrate, benzyl benzoate and benzyl acetate.

### PERSONAL CARE COMPOSITIONS

In preferred methods of the present invention the effective amount of 1,1-dimethoxy-2,2,5-trimethyl-4-hexen is part of a personal care composition.

In preferred methods of the present invention a sensory complex is used (as defined above), the sensory complex preferably being part of a personal care composition.

In preferred methods of the present invention, when the effective amount of fragrance material above is part of a personal care composition, said effective amount of 1,1-dimethoxy-2,2,5-trimethyl-4-hexen is in the range of from 0.01 to 2%, preferably in the range of from 0.05 to 1%, based on the total weight of the personal care composition.

In further preferred methods of the present invention, when a sensory complex as described above is part of a personal care composition, the total amount of the sensory complex is in the range of from 0.05 to 10%, preferably in the range of from 0.2 to 2%, based on the total weight of the personal care composition.

The sensory complex used in the methods of the present invention may be produced by blending
(i) 1,1-dimethoxy-2,2,5-trimethyl-4-hexen, and
(ii) one or more fragrance compounds different from the compounds of group (a), preferably selected from group (B), and optionally
(iii) one or more fragrance compounds selected from the group consisting of tetrahydrogeraniol, methyl dihydro jasmonate, 1-menthol, n-decanal, diethyl malonate, and anethol,
(iv) one or more essential oils, selected from group (V), and/or
(v) one or more cosmetically acceptable diluents, preferably selected form group (D)
under ambient conditions until the final mixture is homogenous, using equipment and methodology commonly known in the art of fragrance compounding.

It is preferable to store the final sensory complex mixture under ambient conditions for a few hours after mixing before using it as a component of a personal care composition.

Personal care compositions used in preferred methods of the present invention may be produced by blending the desired components with the sensory complex using equipment and methodology commonly known in the art of personal care product manufacture. In order to improve the solubilization of the sensory complex in aqueous personal care compositions, the sensory complex may be pre-blended with one or more of the nonionic surfactants.

Personal care composition for use in the methods of the present invention refer to personal cosmetic, toiletry, and healthcare products and are preferably selected from the group consisting of leave-on products, rinse-off products, oral care products, oral care devices, air-care products or air-care devices, such as wipes, washes, baths, shampoos, gels, soaps, sticks, balms, sachets, pillows, mousses, sprays, lotions, creams, cleansing compositions, powders, oils, bath oils and other bath compositions which may be added to a bath.

Personal care compositions may also include, but are not limited to, aerosols, candles, and substances that may be used with vaporizers. The aforementioned wipes, washes, baths, shampoos, gels, soaps, sticks, balms, sachets, pillows, mousses, sprays, lotions, creams, cleansing compositions, oils, bath oils, aerosols, candles and substances which may be used with vaporizers are commercially known to those who have a knowledge of preparing personal care compositions.

Preferred leave-on products for use in the methods of the invention are selected from the group consisting of wipes, gels, sticks, mousses, sprays, lotions, creams, facial masks, cleansing compositions, powders, and oils.

Preferred rinse-off products for use in the methods of the invention are selected from the group consisting of wipes, washes, baths, shampoos, gels, soaps, sticks, balms, sachets, pillows, mousses, sprays, lotions, creams, cleansing compositions, powders, oils, and bath oils.

Alternative preferred personal care composition for use in the methods of the invention are selected from the group consisting of air-care products or devices selected from the group consisting of pump-sprays, aerosols, candles, vaporizers, diffusers, wicking systems, fans, piezoelectric systems, heated oil baths, and incense sticks.

Alternative preferred personal care composition for use in the methods of the present invention are oral care products selected from the group consisting of toothpastes, dentifrices, mouth sprays, mouth washes, chewing gums, mouth cleansers, and dental devices.

Personal care compositions for use in the methods of the invention comprising 1,1-dimethoxy-2,2,5-trimethyl-4-hexen or a sensory complex as described above are capable of increasing the heart rate, preferably by 5% to 100%, more preferably by 5 to 50%, most preferably by 10 to 25%.

In addition, the invention includes a personal care composition comprising an effective amount of the sensory complex according to the present invention and a method of energizing, invigorating, activating, stimulating, vivicating, vitalizing or enliving a human being comprising administering to said human being a personal care composition comprising an effective amount of the sensory complex according to the present invention.

One method of energizing a human is via the use of a personal care composition as described above that comprises, based upon the total weight of the personal care composition, from 0.05 to 10%, preferably from 0.20% to 2.5%, more preferably from 0.2 to 2%, most preferably from 0.3% to 1.5%, by weight of a sensory complex according to the present invention.

In order to achieve the desired response in a human being, the personal care composition may be used in a dosing amount that is in accordance with the prescribed directions of the personal care composition.

The personal care composition may be massaged or placed onto the skin of a human being at any time in accordance with the use instructions of the personal care product. This allows the fragrance to be released from the product slowly and enter the nasal cavity of a human.

Further aspects of the present invention are described in the examples and the attached set of claims.

### EXAMPLES

In order to illustrate the invention the following examples are included.

Unless stated otherwise all figures, data and percentages refer to the weight.

### Example 1

Results of the energizing fragrance materials according to group (a) of the present invention

In order to introduce compounds of group (a) or a sensory complex as described above into the nasal cavity that will occur using many types of products containing the sensory complex the following method of human testing and data collection was developed, standardized and used to collect and analyse data to substantiate the energizing effects of the sensory complex materials in group (a).

Data collection and analysis of each panellist's heart rate was made using a finger clip attachment for the Heart Rhythm Monitor using Freeze-Framer^{®} 2.0 software from HeartMath^{®}, 14700 West Park Avenue, Boulder Creek, California.

The test is divided into 3 parts:
1) the pre-sniff-test period: places the panelist in a neutral state (15 min),
2) the sniff-test period (5 min),
3) the post-sniff-test period (10 min). Typically during each test session, three fragrances (single compound or mixture of compounds) are presented to the panellist, so one test lasts for one hour.

The method used involved the following protocol:
- The stimulating effects of the fragrances (single compound or mixture of compounds) tested on 40 healthy human subjects (panelists)
- All subjects have to be non-smokers with a normal body mass index, who are free of allergies to odorants and do not receive any medical treatment at the time of testing
- Male and female subjects aged between 18 and 35 years participated in the study
- Subjects are tested in individual sessions.
- All experiments are conducted in a bright and quiet room
- The ambient temperature should be between 24-26°C
- Upon arrival, the subject is identified through his personal data: name, age, sex and body mass index
- In addition, he is asked about his mental and emotional condition (no persons under medical anti-depressant prescription are permitted).
- Subsequently, panelists are informed about the proceedings
- They are informed that a fragrance will be presented, but are unaware of the kind of fragrance and the exact time of administration
- At 48 hours prior to testing, panellists had to abstain from food, beverages and toiletries containing tea, coffee and alcohol
- Panellists are seated in a semi-reclined position, providing easy access to attach the finger sensor
- During the testing, the panellist mustn't move nor talk
- Panelist can close his or her eyes, think about pleasant things or breathe in and out deeply
- After completion of the interview and rating scales, the heartbeat measurement can start
- A fragrance (single compound or mixture of compounds) or a placebo substance is administered
- Fragrances are diluted in an odourless solvent (e.g. mineral oil) which can also be used as placebo substance
- Dilution of the fragrance: a total of 0.3 wt.-% to 2 wt.-% according to the odor intensity of the fragrance
- The heartbeat before, during and after the inhalation of the fragrance for each panelist is monitored
- Immediately after each test, the panellist will rate the pleasantness of the fragrance through a questionnaire
**Table I:**

| Representative excerpt of heartbeat-data for panellists that evaluated Amarocite^{®} (1,1-dimethoxy-2,2,5-trimethyl-4-hexene) tested as 1 wt.-% solution in mineral oil | | | |
|---|---|---|---|
| **Heartbeat (bpm)** | | | |
| **Panellist** | **Before** | **After** | **Difference** |
| A | 84 | 90 | + 6 |
| B | 68 | 77 | + 9 |
| C | 56 | 81 | + 25 |
| D | 64 | 70 | + 6 |
| E | 68 | 78 | + 10 |
| F | 60 | 72 | + 12 |
| G | 59 | 71 | + 12 |
| **Mean** | **65.6** | **77** | **+ 11.4** |

After administering Amarocite^{®} (1,1-dimethoxy-2,2,5-trimethyl-4-hexene) to the panelists according to the above protocol, an average heart rate increase of 17.3% was observed.

Very similar results were obtained with a solution of a mixture of 0.4 wt.-% of tetrahydrogeraniol and 0.4 wt.-% of aldehyde C10 (tested in mineral oil). *

Similar results were obtained with a solution of a mixture of 0.3 wt.-% of I-menthol and 0.5 wt.-% of aldehyde C10 (tested in mineral oil).*

Stronger energizing effects were observed with 0.8 wt.-% of tetrahydrogeraniol alone or with 2.0 wt.-% methyl dihydro jasmonate alone (both tested as solutions in mineral oil).*
* These embodiments are not part of the invention as claimed.

### Example 2

Fragrances with energizing effect. Only "white tea" and "red grapefruit" can be used in the invention as claimed.

| **COMPOUND** | **Fragrance Type** | | | | |
|---|---|---|---|---|---|
| | **Jasmine** | **White Tea** | **Mint** | **Red Grapefruit** | **Rose** |
| AMAROCITE^{®} | | 3 | | 40 | |
| HEDIONE^{®} | 80 | 400 | | 10 | |
| MENTHOL L | | | 622 | | |
| TETRAHYDROGERANIOL | | | | | 300 |
| AGRUMEX^{®} | 68 | 9 | | | |
| AGRUNITRIL 10% in DPG | | 2 | | | |
| ALDEHYDE C8 | | | | 60 | |
| ALDEHYDE C8 10% in DPG | | 3 | | | |
| ALDEHYDE C9 | | | | 10 | |
| ALDEHYDE C10 | | | | 50 | |
| ALDEHYDE C10 10% in DPG | | 5 | | | |
| ALDEHYDE C11 UNDECYLENIC | | | | 2 | 2 |
| ALDEHYDE C12 LAURIC | | | | 4 | |
| ALDEHYDE C14 SO-CALLED | 8 | | | | |
| ALDEHYDE C14 SO-CALLED 10% in DPG | | 5 | | | |
| ALLYL AMYL GLYCOLATE | 2 | | | | |
| ALLYL CAPROATE | 12 | | | | |
| ALLYL CYCLOHEXYL PROPIONATE | 10 | | | | |
| ALLYL HEPTOATE | 32 | | | | |
| ALLYL IONONE | | 1 | | | |
| AMBROXAN^{®} 10% in DPG | | 4 | | | |
| ANETHOL | 2 | | 1 | | |
| ANISYL ACETATE | | | | 2 | |
| BENZALDEHYDE | | | 2 | | |
| BOURGEONAL^{®} | | | | 6 | |
| CARDAMOM OIL | | 1 | | | |
| CARVONE L | | | | 2 | |
| DAMASCENON MELANGE 1:1 | | | | | 1 |
| DAMASCENONE 10% in DPG | | 5 | | | |
| DAMASCONE DELTA 10% in DPG | | 5 | | | |
| DIHYDROEUGENOL | | | 1 | | 30 |
| DIHYDROMYRCENOL | 85 | | | | |
| DIMETHYL BENZYL CARBINYL ACETATE | 10 | 5 | | | |
| DIMETHYL BENZYL CARBINYL BUTYRAT | 43 | 3 | | | |
| DIPROPYLENE GLYCOL | | 175 | | 686 | |
| ETHYL BUTYRATE | 5 | | | | |
| ETHYL CAPROATE 10% in DPG | | 4 | | | |
| ETHYL LINALOOL | | | | 20 | |
| ETHYL MALTOL 1% in DPG | 2 | | | | |
| ETHYL MALTOL 5% in DPG | | | | 40 | |
| ETHYL METHYL BUTYRATE-2 | 15 | | | | |
| ETHYL VANILLIN | | | | 2 | |
| ETHYLENE BRASSYLATE | 10 | 40 | | | |
| EUCALYPTOL NAT. | | | 20 | | |
| EVERNYL^{®} 10% in DPG | | 4 | | | |
| FLORAZON^{®} | | 1 | | | |
| FREESIOL / CORPS 119 | | 15 | | | |
| GERANIOL | | 6 | | | |
| GERANIUM OIL | | | 1 | | |
| GINGER OIL | | 1 | | | |
| GLOBALIDE^{®} | | 20 | | | |
| GRAPEFRUIT BASE | | | | 10 | |
| HERBAFLORAT^{®} | 36 | | | | |
| HERBYL PROPIONATE | 23 | | | | |
| HEXENOL CIS-3 | | 2 | | 2 | |
| HEXENOL CIS-3 10% in DPG | 21 | | | | |
| HEXENYL ACETATE CIS-3 10% in DPG | 36 | | | | |
| HEXYL ACETATE | 7 | | | | |
| INDOLE 10% in DPG | | 2 | | | |
| IONONE BETA | | 20 | | | |
| ISO E SUPER^{®} | | 30 | | | |
| JASMOPYRANE | 37 | | | | |
| LEMONGRASS OIL | | 3 | | | |
| LIGUSTRAL^{®} | | 4 | | | |
| LILIAL^{®} | | 15 | | | |
| LINALOOL OXIDE | | | | 4 | |
| LINALYL ACETATE | | 95 | | | |
| LINALYL ISOBUTYRATE | | | | 10 | |
| MACROLIDE^{®} 50% in TEC | | | | 6 | |
| MALTOL | | | 1 | | |
| MELONAL^{®} | 3 | | | | |
| MENTHONE L / ISOMENTHONE D ratio 82/18 | | | | | 10 |
| MENTHONE/ISOMENTHONE RAC. | | | 300 | | |
| MENTHYL ACETATE RAC. | | | 30 | | |
| METHYL ANTHRANILATE 1% in DPG | | 10 | | | |
| METHYL HEPTENONE-6,5,2 | | | | 10 | |
| MUGETANOL^{®} | 35 | | | | |
| OCTINIA BASE 10% in DPG | 2 | | | | |
| ORANGE 20X BASE | | | 2 | | |
| ORANGE OIL 5X | | 5 | | | |
| ORANGENAL NAT. | | | | 4 | |
| OXANTHIA 50% in TEC 1% in DPG | 6 | 5 | | | |
| OXANTHIA 50% in TEC | | | | 6 | |
| PEACHOLIDE 10% in DPG | | 4 | | | |
| PHENIRAT^{®} | 76 | | | | |
| PHENOXANOL^{®} | | 30 | | | |
| PHENYLETHYL ACETATE | | | | | 15 |
| PHENYLETHYL ALCOHOL | | 15 | | | |
| PHENYLETHYL ALCOHOL | 77 | | | | 641 |
| PHENYLETHYL PHENYLACETATE | | 3 | | | |
| PRENYL ACETATE | 3 | | | | |
| ROSE OXIDE L | | | | | 1 |
| SPEARMINT OIL | | | 10 | | |
| TETRAHYDROLINALOOL | 90 | 30 | | | |
| TETRAHYDROMUGUOL | 33 | | | | |
| THYME OIL | | | 10 | | |
| THYMOL 10% in DPG | | 5 | | | |
| UNDECAVERTOL^{®} 10% in DPG | 31 | | | | |
| VANILLIN 10% in DPG | | | | 4 | |
| VETIKOL ACETATE 1% in DPG | | 5 | | | |
| **TOTAL** | **900** | **1.000** | **1.000** | **990** | **1.000** |

| | | | | | |
|---|---|---|---|---|---|
| DPG = dipropylene glycol; NAT. = natural; RAC. = racemic; TEC = triethyl citrate | | | | | |

### Example 5

Antiperspirant aerosol according to the present invention with an energizing sensory complex

| **Part** | **% Wt** | **Ingredient** | **Supplier** |
|---|---|---|---|
| 1 | 10.00 | DC Fluid 244 (Cyclomethicone) | Dow Corning |
| 2 | 7.50 | Micro Dry (Aluminum Chlorohydrate) | Reheis |
| 3 | 1.25 | Myritol PC (Propylene Glycol Dicaprylate/Dicaprate) | Cognis Care |
| 4 | 0.75 | Fragrance Type "White Tea" | Example 2 |
| 5 | 80.0 | Hydrocarbon Propellant A-46 | |

Compounding Procedure: combine parts 1 - 4 in a suitable vessel. Mix until a homogeneous dispersion is achieved. Fill into aerosol cans, crimp on valve and pressure fill propellant (part 5) (a 20/80 blend of propane/isobutane).

### Example 6

Antiperspirant stick according to the present invention with an energizing sensory complex

| **Composition** | **6.1** |
|---|---|
| Phenyl Trimethicon (SilCare TM Silicone 15 M 50) | 13.50 |
| Cetearyl alcohol | To 100 |
| Cetiol CC (dicaprylyl carbonate) | 13.50 |
| Stearic acid | 3.50 |
| PEG-40-hydrated castor oil (Emulsogen TM HCO 040) | 4.10 |
| PEG-8 distearate (Cithrol 4 DS) | 4.10 |
| Petrolatum | 6.90 |
| Aluminum hydrochlorate | 13.80 |
| Aluminum zirconium trichlorohydrex gly | 19.50 |
| Neo Heliopan^{®} Hydro (phenylbenzimidazole sulphonic acid | 0.50 |
| 2,2-dimethyl-3-phenylpropanol | 0.25 |
| Ethylhexylglycerine (octoxyglycerin) | 0.30 |
| 1,1-dimethyl-3-phenylpropanol | 0.25 |
| Fragrance Type "Rose" of Example 2 | - |
| Fragrance Type "Red Grapefruit" of Example 2 | 0.75 |

### Example 7

Suspension roll-on according to the present invention with an energizing sensory complex

| **Composition** | **7.1** | **7.2** |
|---|---|---|
| Silicone | To 100 | To 100 |
| Ethylhexylglycerine (octoxyglycerin) | 1.00 | 1.00 |
| Quaternium-18 hectorite | 13.00 | 13.20 |
| Aluminum hydrochlorate, powder | 21.00 | 20.00 |
| 1,1-dimethyl-3-phenylpropanol | 0.25 | 0.50 |
| 4-methyl-4-phenyl-2-pentanol | 0.10 | - |
| Fragrance Type "White Tea" of Example 2 | 0.25 | - |
| Fragrance Type "Red Grapefruit" of Example 2 | - | 0.85 |
| Fragrance Type "Jasmine" of Example 2 | 0.50 | - |

### Example 8

Suspension stick according to the present invention with an energizing sensory complex

| **Composition** | **8.1** |
|---|---|
| Stearyl alcohol | 20.00 |
| Cyclomethicone | To 100 |
| PPG-14 butyl ether | 2.00 |
| Hydrated castor oil | 1.00 |
| Talc | 2.00 |
| Aluminum hydrochlorate, powder | 20.00 |
| Triclosan® (5-chloro-2-(2,4-dichlorphenoxy)phenol) | 0.30 |
| Ethylhexylglycerine (octoxyglycerin) | 0.50 |
| 1,1-dimethyl-3-phenylpropanol | 0.30 |
| 2,2-dimethyl-3-phenylpropanol | 0.30 |
| Fragrance Type "White Tea" of Example 2 | 0.35 |
| Fragrance Type "Red Grapefruit" of Example 2 | 0.50 |
| Fragrance Type "Jasmine" of Example 2 | - |
| Fragrance Type "Mint" of Example 2 | - |

### Example 11

O/W lotion according to the present invention with an energizing sensory complex

| **Composition** | **11.1** |
|---|---|
| Paraffin oil | 5.00 |
| Isopropylpalmitate | 5.00 |
| Cetyl alcohol | 2.00 |
| Beeswax | 2.00 |
| Ceteareth-20 | 2.00 |
| PEG-20-glyceryl stearate | 1.50 |
| Glycerine | 3.00 |
| Phenoxy ethanol | 0.50 |
| 1,2-Pentanediol | - |
| Parabens (mixture of methyl-, ethyl-, propyl-, butyl-, isobutylparaben) | - |
| Fragrance Type "Jasmine" of Example 2 | - |
| Fragrance Type "White Tea" of Example 2 | 0.90 |
| Fragrance Type "Rose" of Example 2 | - |
| Water | To 100 |

### Example 13

Mouthwash concentrate according to the present invention with an energizing sensory complex

| **Composition** | **13.1** | **13.2** |
|---|---|---|
| Ethanol, 95 % strength | 80.00 | 80.00 |
| Na cyclamate | 0.15 | 0.15 |
| Eucalyptol aroma (contains natural eucalyptol) | - | 1.00 |
| Dyestuff | 0.01 | 0.01 |
| Fragrance Type "Mint" of Example 2 | 1.00 | - |
| Fragrance Type "White Tea" of Example 2 | 2.50 | 3.00 |
| Dist. water | Ad 100.00 | Ad 100.00 |

### Example 14

Dental cream according to the present invention with an energizing sensory complex

| **Compositions** | **14.1** | **14.2** |
|---|---|---|
| Carrageenan | 0.90 | 0.90 |
| Glycerol | 15.00 | 15.00 |
| Sorbitol 70 %, in water | 25.00 | 25.00 |
| PEG 1000 | 3.00 | 3.00 |
| Na-fluoride | 0.24 | 0.24 |
| Tetrapotassium diphosphate | 4.50 | 4.50 |
| Tetrasodium diphosphate | 1.50 | 1.50 |
| Na saccharinate | 0.40 | 0.40 |
| Precipitated silica | 20.00 | 20.00 |
| Titanium dioxide | 1.00 | 1.00 |
| p-Hydroxybenzoic acid methyl ester | 0.10 | 0.10 |
| Fragrance Type "Red Grapefruit" of Example 2 | 1.00 | - |
| Fragrance Type "White Tea" of Example 2 | - | 1.25 |
| Fragrance Type "Mint" of Example 2 | 0.50 | - |
| Sodium dodecyl sulfate | 1.30 | 1.30 |
| Dist. water | Ad 100.00 | Ad 100.00 |

### Example 16

Dental gel according to the present invention with an energizing sensory complex

| **Composition** | **16.1** |
|---|---|
| Na-carboxymethylcellulose | 0.40 |
| Sorbitol 70 %, in water | 72.00 |
| Polyethylene glycol (PEG) 1500 | 3.00 |
| Na-saccharinate | 0.07 |
| Na-fluoride | 0.24 |
| p-Hydroxybenzoic acid (PHB) ethyl ester | 0.15 |
| Abrasive silica | 11.00 |
| Thickening silica | 6.00 |
| Triclosan (2,4,4'-trichlor-2'-hydroxydiphenyl ether) | 0.50 |
| Cooling agent WS-3 | 0.10 |
| Fragrance Type "White Tea" of Example 2 | 1.10 |
| Fragrance Type "Mint" of Example 2 | - |
| Sodium dodecyl sulfate (SDS) | 1.40 |
| Dist. water | Ad 100.00 |

### Example 17

Skin oil according to the present invention with an energizing sensory complex

| **Composition** | **17.1** |
|---|---|
| Cetyl palmitate | 3.00 |
| C₁₂₋₁₅- Alkyl benzoate | 2.00 |
| Polyisobutene | 10.00 |
| Squalane | 2.00 |
| Fragrance Type "White Tea" of Example 2 | 0.50 |
| Fragrance Type "Jasmine" of Example 2 | - |
| Fragrance Type "Rose" of Example 2 | - |
| Preservative | q.s. |
| Mineral oil | ad 100 |

### Example 18

Shampoo according to the present invention with an energizing sensory complex

| **Composition** | **18.1** |
|---|---|
| Sodium laurylether sulfate (Texapon NSO, Cognis) | 20.00 |
| Cocamidopropyl betaine (Dehyton K, Cognis) | - |
| Sodium chloride | 1.40 |
| Citric acid | 1.30 |
| Phenoxyethanol | 0.30 |
| Methyl-, Ethyl-, Butyl-, and Propyl parabenes | 0.30 |
| Fragrance Type "White Tea" of Example 2 | 0.50 |
| Fragrance Type "Jasmine" of Example 2 | - |
| Fragrance Type "Rose" of Example 2 | - |
| Water | Ad 100 |

### Example 19

Air freshener gel according to the present invention with an energizing sensory complex

| **Composition** | | 19.1 |
|---|---|---|
| Water | | Ad 100 |
| Genugel^{®} X-6424 | Carrageenan | 2.00 |
| Arkopal^{®} N 100 | Emulsifier | 3.20 |
| Preventol^{®} D 7 | Preservative | 5.00 |
| Fragrance Type " Jasmine " of Example 2 | Perfume | - |
| Fragrance Type "Red Grapefruit" of Example 2 | Perfume | 1.00 |
| Fragrance Type "Rose" of Example 2 | Perfume | - |

### Example 20

Deodorant sticks according to the present invention with an energizing sensory complex

| **Composition** | **20.1** |
|---|---|
| Sodium stearate | 8.00 |
| 1,2-Hexanediol | 0.50 |
| 1,2-Octanediol | - |
| 1,2-Propylene glycol | 41.00 |
| Butylene glycol | 5.00 |
| 2-Hexyldecanoic acid | 0.50 |
| 2-Butyoctanoic acid | - |
| Ceteareth-25 | 3.00 |
| Ethanol | 18.00 |
| Fragrance Type "White Tea" of Example 2 | 0.25 |
| Fragrance Type "Jasmine" of Example 2 | - |
| Fragrance Type "Rose" of Example 2 | - |
| Water | Ad 100 |

## Claims

1. A non-therapeutic method of energizing a human being comprising the following steps:
- providing an effective amount of a fragrance material comprising or consisting of-1,1-dimethoxy-2,2,5-trimethyl-4-hexene and
- administering said effective amount of fragrance material to said human being in a way that said amount of fragrance material reaches the nasal cavity of said human being,
wherein said administered amount of fragrance material is capable of increasing the heart rate of said human being based on the heart rate of said human being before administration of said amount of fragrance material.

2. A method according to claim 1 wherein said fragrance material comprises at least one additional fragrance selected from the group consisting of tetrahydrogeraniol, methyl dihydro jasmonate, 1-menthol, n-decanal, diethyl malonate, and anethol.

3. A method according to claims 1 or 2, wherein 1,1-dimethoxy-2,2,5-trimethyl-4-hexene is part of a sensory complex, wherein the sensory complex comprises or consists of:
(i) an effective amount of 1,1-dimethoxy-2,2,5-trimethyl-4-hexen and additionally
(ii) one or more fragrance compounds different from the compounds of group (i) and (iii) as defined below
(iv) one or more essential oils, and/or
(v) one or more cosmetically acceptable diluents and optionally (iii) one or more fragrance compounds selected from the group consisting of tetrahydrogeraniol, methyl dihydro jasmonate, 1-menthol, n-decanal, diethyl malonate, and anethol.

4. A method according to any preceding claim 1 to 3, wherein said effective amount of 1,1-dimethoxy-2,2,5-trimethyl-4-hexene is part of a personal care composition.

5. A method according to claim 3, wherein said sensory complex is part of a personal care composition.

6. A method according to claim 4, wherein said effective amount of 1,1-dimethoxy-2,2,5-trimethyl-4-hexene is in the range of from 0.01 to 2 percent based on the total weight of the personal care composition.

7. A method according to claim 5 wherein the total amount of said sensory complex is in the range of from 0.05 to 10 percent based on the total weight of the personal care composition.

8. A method according to any of claims 4 to 7, wherein the personal care composition is selected from the group consisting of leave-on products, rinse-off products, oral care products, oral care devices, air-care products or air-care devices.

9. A method according to claim 8, wherein the personal care composition is a leave-on product selected from the group consisting of wipes, gels, sticks, mousses, sprays, lotions, creams, facial masks, cleansing compositions, powders, and oils.

10. A method according to claim 8, wherein the personal care composition is a rinse-off product selected from the group consisting of wipes, washes, baths, shampoos, gels, soaps, sticks, balms, sachets, pillows, mousses, sprays, lotions, creams, cleansing compositions, powders, oils, and bath oils.

11. A method according to claim 8, wherein the personal care composition is an air-care product or device selected from the group consisting of pump-sprays, aerosols, candles, vaporizers, diffusers, wicking systems, fans, piezoelectric systems, heated oil baths, and incense sticks.

12. A method according to claim 8, wherein the personal care composition is an oral care product or device selected from the group consisting of toothpastes, dentifrices, mouth sprays, mouth washes, chewing gums, mouth cleansers, and dental devices.

## Patentansprüche

1. Nicht-therapeutisches Verfahren zur Antriebssteigerung bei einem menschlichen Lebewesen, das die folgenden Schritte umfasst:
- Zuführung einer wirksamen Menge eines Duftstoffes enthaltend oder bestehend aus 1,1-Dimethoxy-2,2,5-trimethyl-4-hexen und
- Verabreichung dieser wirksamen Menge eines Duftstoffes an das besagte menschliche Lebewesen in einer solchen Weise, dass die Duftstoffmenge den Nasenhöhlenraum des menschlichen Lebewesens erreicht,
wobei besagte verabreichte Duftstoffmenge ausreichend ist, um den Herzschlags des menschlichen Lebewesens ausgehend vom Herzschlag des menschlichen Lebewesens vor Verabreichung der besagten Duftstoffmenge zu steigern.

2. Verfahren nach Anspruch 1, wobei besagte Duftstoffmenge wenigstens einen weiteren Duftstoff enthält, welcher ausgewählt ist aus der Gruppe, die gebildet wird von Tetrahydrogeraniol, Methyldihydrojasmonat, 1-Menthol, n-Decanal, Dimethylmalonat und Anethol.

3. Verfahren nach den Ansprüchen 1 oder 2, wobei das 1,1-Dimethoxy-2,2,5-trimethyl-4-hexen Teil eines Sensorikkomplexes ist, wobei dieser Sensorikkomplex aus folgenden Komponenten besteht:
(i) einer wirksame Menge von 1,1-Dimethoxy-2,2,5-trimethyl-4-hexen und zusätzlich
(ii) einem oder mehreren Duftstoffen, die von den Stoffen der Gruppe (i) verschieden sind,
sowie gegebenenfalls
(iii) einen oder mehreren Duftstoffen, die ausgewählt sind aus der Gruppe, die gebildet wird von Tetrahydrogeraniol, Methyldihydrojasmonat, 1-Menthol, n-Decanal, Dimethylmalonat und Anethol,
(iv) einem oder mehreren essentiellen Ölen, und/oder
(v) einem oder mehreren kosmetisch verträglichen Lösungsmitteln.

4. Verfahren nach einem der vorhergehenden Ansprüche 1 bis 3, wobei die besagte wirksame Menge an 1,1-Dimethoxy-2,2,5-trimethyl-4-hexen Bestandteil eines Körperpflegemittels ist.

5. Verfahren nach Anspruch 3, wobei der besagte Sensorikkomplex Bestandteil eines Körperpflegemittels ist.

6. Verfahren nach Anspruch 4, wobei die besagte wirksame Menge an 1,1-Dimethoxy-2,2,5-trimethyl-4-hexen im Bereich von 0,01 bis 2 % bezogen auf die Gesamtmenge des Körperpflegemittels liegt.

7. Verfahren nach Anspruch 5, wobei die Gesamtmenge des besagten Sensorikkomplexes im Bereich von 0,05 bis 10 % bezogen auf die Gesamtmenge des Körperpflegemittels liegt.

8. Verfahren nach einem der Ansprüche 4 bis 7, wobei das Körperpflegemittel ausgewählt ist aus der Gruppe, die gebildet wird von Aufziehprodukten, Auswaschprodukten, Mundpflegemitteln, Mundpflegevorrichtungen, Luftbehandlungsmitteln sowie Vorrichtungen zur Luftbehandlung.

9. Verfahren nach Anspruch 8, wobei das Körperpflegemittel ein Aufziehprodukt darstellt, welches ausgewählt ist aus der Gruppe, die gebildet wird von Tüchern, Gelen, Schäumen, Sprays, Lotionen, Cremes, Gesichtsmasken, Reinigungsmitteln, Pudern und Ölen.

10. Verfahren nach Anspruch 8, wobei das Körperpflegemittel ein Auswaschprodukt darstellt, welches ausgewählt ist aus der Gruppe, die gebildet wird von Tüchern, Wäschen, Bädern, Shampoos, Gelen, Seifen, Stiften, Balsamen, Duftkissen, Kissen, Schäumen, Sprays, Lotionen, Cremes, Reinigungsmitteln, Pudern, Ölen und Badeölen.

11. Verfahren nach Anspruch 8, wobei das Körperpflegemittel ein Luftbehandlungsmittel oder eine entsprechende Vorrichtung darstellt, das ausgewählt ist aus der Gruppe, die gebildet wird von Pumpsprays, Aerosolen, Kerzen, Verdampfern, Diffusoren, Dochtsystemen, Gebläsen, piezoelektrischen Systemen, erwärmten Ölbädern und Räucherstäbchen.

12. Verfahren nach Anspruch 8, wobei das Körperpflegemittel ein Mundpflegeprodukt darstellt, welches ausgewählt ist aus der Gruppe, die gebildet wird von Zahnpasten, Zahnputzmitteln, Mundsprays, Mundspülungen, Kaugummis, Mundreinigungsmitteln und Dentalvorrichtungen.

## Revendications

1. Méthode non thérapeutique destinée à redynamiser un être humain, comprenant les étapes suivantes :
- fournir une quantité efficace d'un matériau de parfum comprenant, ou constitué de, 1,1-diméthoxy-2,2,5-triméthyl-4-hexène, et
- administrer ladite quantité efficace de matériau de parfum audit être humain, de telle sorte que ladite quantité de matériau de parfum atteigne la cavité nasale dudit être humain,
dans laquelle ladite quantité administrée de matériau de parfum est capable d'augmenter la fréquence cardiaque dudit être humain par rapport à la fréquence cardiaque dudit être humain avant l'administration de ladite quantité de matériau de parfum.

2. Méthode selon la revendication 1, dans laquelle ledit matériau de parfum comprend au moins un parfum supplémentaire choisi dans le groupe constitué par le tétrahydrogéraniol, le dihydrojasmonate de méthyle, le 1-menthol, le n-décanal, le malonate de diéthyle, et l'anéthol.

3. Méthode selon les revendications 1 ou 2, dans laquelle le 1,1-diméthoxy-2,2,5-triméthyl-4-hexène fait partie d'un complexe sensoriel, où le complexe sensoriel comprend, ou est constitué de,
(i) une quantité efficace de 1,1-diméthoxy-2,2,5-triméthyl-4-hexène et de plus
(ii) un ou plusieurs composés de parfum différents des composés du groupe (i) et (iii) tel que défini ci-après,
(iv) une ou plusieurs huiles essentielles, et/ou
(v) un ou plusieurs diluants acceptables sur le plan cosmétique,
et éventuellement
(iii) un ou plusieurs composés de parfum choisis dans le groupe constitué par le tétrahydrogéraniol, le dihydrojasmonate de méthyle, le 1-menthol, le n-décanal, le malonate de diéthyle, et l'anéthol.

4. Méthode selon l'une quelconque des revendications précédentes 1 à 3, dans laquelle ladite quantité efficace de 1,1-diméthoxy-2,2,5-triméthyl-4-hexène fait partie d'une composition de soins personnels.

5. Méthode selon la revendication 3, dans laquelle ledit complexe sensoriel fait partie d'une composition de soins personnels.

6. Méthode selon la revendication 4, dans laquelle ladite quantité efficace de 1,1-diméthoxy-2,2,5-triméthyl-4-hexène se trouve dans la plage allant de 0,01 à 2% sur la base du poids total de la composition de soins personnels.

7. Méthode selon la revendication 5, dans laquelle la quantité totale dudit complexe sensoriel se trouve dans la plage allant de 0,05 à 10% sur la base du poids total de la composition de soins personnels.

8. Méthode selon l'une quelconque des revendications 4 à 7, dans laquelle la composition de soins personnels est choisie dans le groupe constitué par les produits sans rinçage, les produits à rincer, les produits de soin oral, les dispositifs de soin oral, les produits de traitement de l'air, ou les dispositifs de traitement de l'air.

9. Méthode selon la revendication 8, dans laquelle la composition de soins personnels est un produit sans rinçage choisi dans le groupe constitué par les lingettes, les gels, les bâtonnets, les mousses, les sprays, les lotions, les crèmes, les masques pour le visage, les compositions de nettoyage, les poudres et les huiles.

10. Méthode selon la revendication 8, dans laquelle la composition de soins personnels est un produit à rincer choisi dans le groupe constitué par les lingettes, les badigeons, les bains, les shampooings, les gels, les savons, les bâtonnets, les baumes, les sachets, les oreillers, les mousses, les sprays, les lotions, les crèmes, les compositions de nettoyage, les poudres, les huiles et les huiles de bain.

11. Méthode selon la revendication 8, dans laquelle la composition de soins personnels est un produit ou un dispositif de traitement de l'air choisi dans le groupe constitué par les sprays à pompe, les aérosols, les bougies, les vaporisateurs, les diffuseurs, les systèmes à mèche, les ventilateurs, les systèmes piézoélectriques, les bains d'huile chauffée, et les bâtons d'encens.

12. Méthode selon la revendication 8, dans laquelle la composition de soins personnels est un produit ou un dispositif de soin oral choisi dans le groupe constitué par les pâtes dentaires, les dentifrices, les sprays buccaux, les rince-bouche, les chewing-gums, les bains de bouche, et les dispositifs dentaires.
